# EUROPÄISCHE PATENTSCHRIFT

(11) **EP 0 704 208 B1**
(45) Veröffentlichungstag und Bekanntmachung des Hinweises auf die Patenterteilung: **03.03.2004**
(21) Anmeldenummer: 95113468.3
(22) Anmeldetag: 28.08.1995
(51) Int. Cl.: A61K 9/32, A61K 9/52, C08F 220/18

(54) **Überzugs- und Bindemittel für Arzneiformen sowie damit hergestellte Arzneiform**
Coating and binder compositions for pharmaceutical forms and pharmaceutical forms prepared therewith
Compositions d'enrobage et de liant pour des formes galéniques et des formes galéniques préparées avec ces compositions

(30) Priorität: 31.08.1994 DE 9414066 U
(43) Veröffentlichungstag der Anmeldung: 03.04.1996
(73) Patentinhaber: Röhm GmbH & Co. KG, 64293 Darmstadt (DE)
(72) Erfinder: Lehmann, Klaus, Dr., D-64380 Rossdorf (DE); Süfke, Thomas, Dr., D-64380 Rossdorf (DE)

(56) Entgegenhaltungen:
- EP-A- 0 386 967
- GB-A- 2 174 599
- US-A- 4 720 387
- DATABASE WPI Section Ch, Week 198414 Derwent Publications Ltd., London, GB; Class A96, AN 1984-084885 XP002134261 & JP 59 034153 A (KOKURITSU EISEI SHI), 24. Februar 1984 (1984-02-24)
- FUJIMURA M., ET AL.: "Preparation of properties of soluble-insoluble immobilized proteases" BIOTECHNOL. BIOENG., Bd. 29, Nr. 6, 1987, Seiten 747-752, XP000882912

## Beschreibung

Die Erfindung betrifft Überzugs- und Bindemittel für Arzneiformen, enthaltend ein Copolymerisat aus äthylenisch ungesättigten, radikalisch polymerisierbaren Monomeren mit einer Carboxylgruppe und Alkylestern solcher Monomerer, das vorzugsweise als Latex in einer wäßrigen Phase dispergiert vorliegt, sowie die Verwendung des Überzugs- und Bindemittels und damit hergestellte Arzneiformen.

### Stand der Technik

Aus der DE-C 2 135 073 sind Überzugsmittel für Arzneiformen mit der oben genannten Zusammensetzung in Form einer wäßrigen Dispersion bekannt. Sie enthalten Emulsionspolymerisate, die in der Regel aus 50 Gew.-% Methacrylsäure und 50 Gew.-% Methyl- oder Ethylacrylat aufgebaut sind. Ein Bindemittel dieser Art hat unter der Handelsbezeichnung EUDRAGIT L30D (Warenzeichen der Röhm GmbH, Darmstadt) wirtschaftliche Bedeutung erlangt.

Daraus hergestellte Arzneimittelüberzüge sind im sauren Milieu des Magensaftes unlöslich. Sie gehen erst im Darmsaft bei einem pH-Wert von 5,5 und mehr in Lösung und geben dann den wirkstoffhaltigen Kern zur Auflösung frei. Für verschiedene Wirkstoffe sind unterschiedliche Freisetzungs-Charakteristiken erwünscht. Manche Arzneiformen sollen den Wirkstoff sofort nach dem Eintritt der Arzneiform in das Duodenum oder die oberen Darmabschnitte bei pH-Werten von etwa 5 bis 6, andere erst in den unteren Darmbereichen bis zum Colon bei pH-Werten von etwa 6 bis 7,5 freisetzen. Für diesen Zweck ist eine ausreichend breite Palette von Überzugsmitteln für verschiedene Freisetzungs-pH-Werte erwünscht, um die Freisetzung der Wirkstoffe in diesen Darmabschnitten zu steuern.

Die Freisetzungs-Charakteristik von Arzneimittelüberzügen in vitro wird nach USP üblicherweise mit künstlichem Magensaft (0,1 n HCl) und künstlichem Darmsaft (pH 6,8) geprüft. Um die abgestufte Auflösung im Darmbereich zu erfassen, hat es sich als zweckmäßig erwiesen, die Freisetzungsgeschwindigkeit eines wasserlöslichen Inhaltsstoffes aus der Arzneiform zunächst innerhalb von 2 Stunden in künstlichem Magensaft und anschließend in Pufferlösungen zu bestimmen und, beginnend bei pH 5,0, alle 60 Minuten die Pufferlösung zu wechseln, wobei der pH-Wert stufenweise um jeweils 0,5 erhöht wird.

Aus EP-B 152 038 sind bereits wäßrige Überzugsmittel bekannt, die auch die Herstellung von Arzneiformen mit einem Freisetzungs-pH-Wert von 7 bis 7,5 gestatten. Dazu ist jedoch die Abmischung von wenigstens zwei Überzugsmitteldispersionen erforderlich, von denen eine für die pH-Steuerung und die andere für die Einstellung der Elastizität verantwortlich sind. Diese neutrale Polymerisatkomponente ist jedoch im Darmsaft unlöslich und führt zu einer häufig unerwünschten Verzögerung der Auflösung, auch wenn der erwünschte pH-Wert schon erreicht ist.

### Aufgabe und Lösung

Der Erfindung liegt die Aufgabe zugrunde, ein Überzugsmittel bereitzustellen, das in Form einer wäßrigen Dispersion einsetzbar ist und Überzüge ergibt, die sich in den tieferen Darmabschnitten oder im Colon auflösen und bei der oben angegebenen Prüfmethode aus überzogenen Arzneiformen eine Wirkstoff-Freisetzung erst ab pH 6,5 bis 7,0 zulassen und spätestens bei pH 7,5 eine Freisetzung innerhalb von -120 Minuten erreichen lassen. Die vorteilhaften Anwendungseigenschaften der bekannten wäßrigen Überzugsmittel sollten dabei erhalten bleiben. So soll beim Trocknen des Überzugs, der nicht mehr als 10 Gew.-% Weichmacher, bezogen auf das Emulsionspolymerisat enthält, eine Filmbildung unter 30°C erfolgen.

Die gestellte Aufgabe ließ sich, ausgehend von einem handelsüblichen Überzugsmittel auf Basis eines Copolymerisats aus Methacrylsäuren und Ethylacrylat im Verhältnis 50 : 50, durch Verminderung des Methacrylsäure-Anteils in dem Copolymerisat nicht lösen; man erreicht dadurch nur eine zeitliche Verzögerung der Auflösung, also einen flacheren Verlauf der Freisetzungskurve, nicht jedoch eine Heraufsetzung des pH-Wertes beim Freigabebeginn. Die Freisetzung setzt selbst bei einem Methacrylsäuregehalt von nur 10 % bei pH 5 bis 6 ein. Auch die Verwendung von Comonomeren, die die Hydrophilie des Überzugs herabsetzen, wie Butyl- oder 2-Ethylhexylester der Acryl- oder Methacrylsäure, führte nicht zum Erfolg, weil auch in diesem Fall nur die Auflösung verzögert, aber der Auflösungsbeginn nicht zu höheren pH-Werten verschoben wird.

Es wurde gefunden, daß die gestellte Aufgabe durch Überzugs- und Bindemittel für Arzneiformen auf Basis eines Copolymerisats aus äthylenisch ungesättigten, radikalisch polymerisierbaren Monomeren mit einer Carboxylgruppe und Alkylestern solcher Monomerer gelöst wird, wenn das Copolymerisat aus
A) 10 bis 25 Gew.-% Methacrylsäure,
B) 40 bis 70 Gew.-% Methylacrylat
C) 20 bis 40 Gew.-% Methylmethacrylat
aufgebaut ist. Vorzugsweise beträgt der Anteil A 10 bis 20 Gew.-% Methacrylsäure.

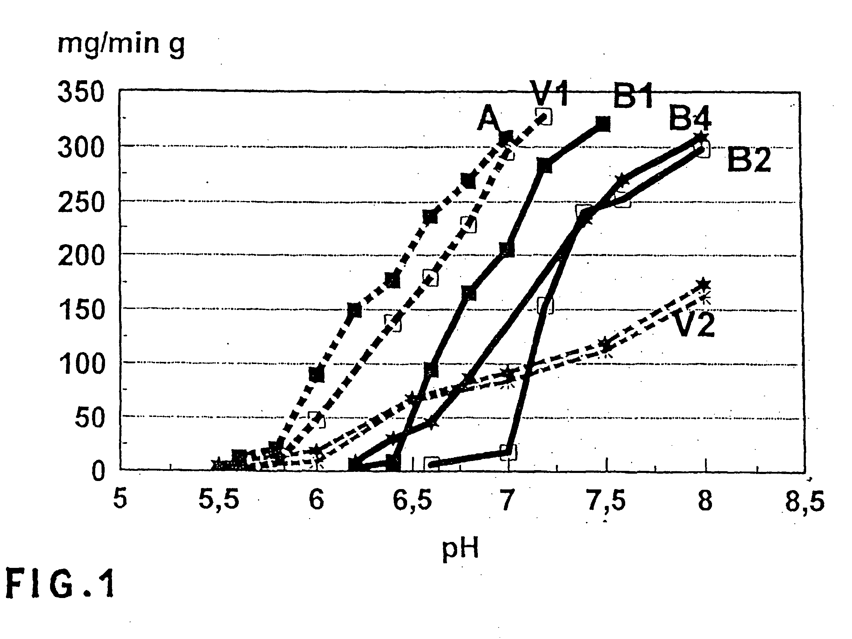

Überraschenderweise setzen Arzneiformen mit Überzügen dieser Zusammensetzung, die nach dem Test gemäß USP magensaftresistent sind, bei pH 6,8 noch praktisch keinen Wirkstoff frei, während schon bei pH 7,5 eine vollständige Freisetzung innerhalb von 60 Minuten erreicht wird. Die vorteilhafte Auflösungscharakteristik der erfindungsgemäßen Überzüge geht aus Figur 1 anschaulich hervor. Hierzu wurden Überzugsfilme in der für Arzneiformen üblichen Schichtdicke auf Glasperlen aufgetragen und durch pH-Stat-Titration mit Alkali die bei bestimmten pH-Werten in Lösung gehende Polymerisatmenge bestimmt.

In Figur 1 sind die Auflösungskurven für drei erfindungsgemäße Überzugsmittel (B1, B2, B4 entsprechend den Ausführungsbeispielen 1, 2 und 4) der Zusammensetzung (in Gew.-%)

| | B1 | B2 | B4 |
|---|---|---|---|
| Methylacrylat | 60 | 65 | 70 |
| Methylmethacrylat | 25 | 25 | 20 |
| Methacrylsäure | 15 | 10 | 10 |

mehreren Überzugsmitteln (A, V1 bis V4) mit den Zusammensetzungen (in Gew.-%):

| | A | V1 | V2 | V3 | V4 |
|---|---|---|---|---|---|
| Methylacrylat | - | 50 | - | - | - |
| Ethylacrylat | - | - | 50 | 65 | 70 |
| Methylmethacrylat | 50 | 20 | 20 | - | - |
| Methacrylsäure | 50 | 30 | 30 | 35 | 30 |

gegenübergestellt. A entspricht dem Handelsprodukt EUDRAGIT L100. Die Herstellung von V1 ist in Beispiel 3 beschrieben. V2, V3 und V4 wurden in entsprechender Weise durch Emulsionspolymerisation erzeugt. Die Kurven für V2, V3 und V4 sind nahezu deckungsgleich und in Figur 1 gemeinsam mit V2 bezeichnet. Der zu höheren pH-Werten verschobene, vorteilhaft steile Kurvenverlauf bei den erfindungsgemäßen Überzugsmitteln B1, B2 und B4 war nicht vorhersehbar. Oberhalb pH 7,5 wird eine vollständige Auflösung des Überzugs erreicht.

Ein weiterer Vorteil liegt in der hohen Elastizität und Dehnbarkeit des Überzugsfilms, die durch den vergleichsweise geringen Anteil an hartmachenden Monomerbestandteilen, wie Methylmethacrylat und Methacrylsäure, begründet sind. Es war allerdings überraschend, daß allein mit Methylacrylat, das von allen Alkylacrylaten als Homopolymerisat die größte Polymerisathärte ergibt, eine den praktischen Anforderungen genügende Dehnbarkeit erreicht wird. Man kommt dadurch im allgemeinen mit Weichmacheranteilen unter 10 Gew.-% oder ganz ohne Weichmacher aus. Dadurch werden fertigungstechnische Nachteile vermieden, die beim Einsatz größerer Mengen an Weichmachern aufzutreten pflegen.

Durch die Erfindung wird eine Lücke in der galenischen Palette der Überzugsmittel mit Freisetzungskurven bei unterschiedlichen pH-Werten geschlossen. Während Überzugsmittel für den Freisetzungsbeginn ab pH 5,5 bis 6,5 zur Verfügung standen, war ein Freisetzungsbeginn ab pH 7 bisher nicht in befriedigender Weise erreichbar.

### Herstellung des Überzugsmittels

Das erfindungsgemäße Überzugsmittel ist in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren.

Die mit A, B und C bezeichneten Monomerbestandteile bilden in der Regel mehr als 80 Gew.-% und vorzugsweise 100 Gew.-% des Copolymerisats. Der gegebenfalls verbleibende Anteil kann aus anderen Acryl- oder Methacrylmonomeren, insbesondere Alkylestern, wie Ethylacrylat und -methacrylat oder Butylacrylat und -methacrylat bestehen. Das Molekulargewicht liegt im üblichen Bereich, beispielsweise 50.000 bis 300.000 Dalton.

Das Copolymerisat kann nach gängigen Verfahren der radikalischen Polymerisation in Gegenwart radikalbildender Initiatoren und gegebenenfalls Reglern zur Einstellung des Molekulargewicht in Substanz, in Lösung oder in Emulsion hergestellt werden. Bevorzugt ist die Emulsionspolymerisation in wäßriger Phase in Gegenwart wasserlöslicher Initiatoren und (vorzugsweise anionischer) Emulgatoren. Das Emulsionpolymerisat wird vorzugsweise in Form einer 10- bis 50-gew.-prozentigen, insbesondere 30 bis 40-prozentigen wäßrigen Dispersion erzeugt und angewendet. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Verdickung der Überzugsmitteldispersion erwünscht sein sollte. Der Gewichtsmittelwert der Latex-Teilchengröße beträgt in der Regel 40 bis 100 nm, vorzugsweise 50 bis 70 nm, was eine verarbeitungstechnisch günstige Viskosität unter 1000 mPa s gewährleistet.

Die Mindestfilmbildungstemperatur (MFT nach DIN 53 778) liegt für die meisten erfindungsgemäßen Überzugsmittel zwischen 0 und 25°C, so daß die Verarbeitung bei Raumtemperatur ohne Weichmacherzusatz möglich ist. Die Reißdehnung der Filme, gemessen nach DIN 53 455, liegt bei einem Gehalt von höchstens 10 Gew.-% Triethylcitrat in der Regel bei 50 % oder mehr.

Die durch DSC-Messung bestimmte Erweichungstemperatur des beim Trocknen entstehenden Polymerisatfilms liegt vorzugsweise im Bereich von 40 bis 80°C, insbesondere von 45 bis 70°C. Die Temperaturstabilität der Emulsionspolymerisate, bestimmt durch thermo-grvimetrische Analyse (Tᵥ0,1) liegt im allgemeinen über 200°C, meist im Bereich von 200 bis 240°C, was für carboxylgruppenhaltige Copolymere überraschend hoch ist. Es ist dadurch auch möglich, die Emulsionspolymerisate aus der Schmelze zu verarbeiten. Typische Schmelzviskositäten liegen beispielsweise für Emulsionspolymerisate aus
60 % MA, 20 % MMA, 20 % MAS bei 8000 Pa s bei 160°C
60 % MA, 25 % MMA, 15 % MAS bei 4000 Pa s bei 150°C
(MA = Methylacrylat, MMA = Methylmethacrylat, MAS = Methacrylsäure, Angaben in Gew.-%)

### Verarbeitung des Überzugsmittels zu Arzneiformen

Die neuen Überzugsmittel können in entsprechender Weise wie andere bekannte wäßrige Überzugsmittel auf Acrylatbasis verarbeitet werden. Am gebräuchlichsten sind das Kesseldragierverfahren und das Wirbelschichtverfahren. Üblichen Zusätzen, wie Weichmachern, Pigmenten, Füllstoffen, Verdickungsmitteln, Entschäumungsmitteln, Konservierungsmitteln usw. können in gebräuchlichen Mengen mitverwendet werden. Überzüge können auf Tabletten, Dragees, Granulaten oder Kristallen erzeugt werden. Auch die Bildung von Matrix-Tabletten oder -Granulaten ist möglich. Bevorzugte Verarbeitungstemperaturen liegen im Bereich von 30 bis 60°C. Geeignete Filmdicken betragen 10 bis 80 Mikrometer.

Nach dem Mechanismus der Wirkstoffabgabe kann die pH-Steuerung mittels des Überzugsfilms nicht nur im Magen-Darm-Trakt, sondern auch in anderen Körperhöhlen, Geweben, Blutbahnen und den Lebensräumen von Tieren und Pflanzen genutzt werden, um dort im Zusammenhang mit einer pH-Änderung die Freisetzung von Wirkstoffen herbeizuführen. Beispiele sind Filme, die mit Kathetern in die Blutbahn eingebracht werden, Implantate von Tierarzneimitteln und Vaccine, die dem Fischfutter beigemischt werden.

Ebenso wie mit anderen wäßrigen Überzugsmitteln können Schichten von mehrlagigen Überzugssystemen erzeugt werden. Beispielsweise kann ein Kern, der z.B. basische oder wasserempfindliche Wirkstoffe enthält, mit einer Isolierschicht aus einem anderen Überzugsmaterial, wie Celluloseether, Celluloseester, kationische Polymethacrylate (wie EUDRAGIT ® E100, -RL100, -RS100, Röhm GmbH), versehen werden, bevor das erfindungsgemäße Überzugsmittel aufgetragen wird. Ebenso können anschließend weitere Überzüge, beispielsweise mit geschmackskaschierender Wirkung oder mit ansprechender Farb- oder Glanzwirkung, aufgebracht werden.

### Beispiel 1 - Überzugsmittel B1

297 g eines Emulsionspolymerisates aus 60 Gew.-Teilen Methylacrylat, 25 Gew.-Teilen Methylmethacrylat und 15 Gew.-Teilen Methacrylsäure mit 30 % Trockensubstanz (89 g Polymersubstanz) wurden durch Zugabe von 238 g Wasser verdünnt. 1000 g runde, schwach gewölbte Tablettenkerne von 7 mm Durchm., 3,3 mm Höhe und 140 mg Gewicht, bestehend aus Laktose (59,2 Gew.-%), Aerosil 200 (0,5 Gew.-%), Talkum (3,0 Gew.-%), mikrokristalliner Cellulose Avicel pH 102 (30 Gew.-%), Magnesiumstearat (0,3 Gew.-%), Amijel (Stärke) (5,0 Gew.-%) sowie Methylenblau (2,0 Gew.-%) als Indikator wurden in einen zwiebelförmigen Dragierkesel von 25 cm Durchmesser unter Rotation von 40 Umdrehungen pro Minute mit Warmluft auf 30 Grad C erwärmt und die Dispersion bei dieser Temperatur mit einer Luftdrucksprühpistole mit 1 bar Druckluft besprüht. Die Dispersion wurde mit einer Sprühgeschwindigkeit von etwa 4 g Dispersion pro Minute innerhalb von 140 min kontinuierlich aufgesprüht. Die überzogenen Tabletten wurden 2 Std. bei 40 Grad C im Trockenschrank nachgetrocknet, über Nacht bei Raumbedingungen offen gelagert und ihr Zerfalls- und Auflösungsverhalten nach USP <711> Dissolution/Apparatur 2 (paddle) geprüft. Die überzogenen Tabletten waren 120 min in künstlichem Magensaft unverändert, auch weitere 60 min nach Abgießen des Magensaftes in Pufferlösung pH 5,0. Mit Pufferlösung pH 6,8 waren alle Tabletten innerhalb von 40 min zerfallen.

### Beispiel 2 - Überzugsmittel B2

Analog zum Beispiel 1 wurden 297 g eines Emulsionspolymerisates aus 65 Gew.-Teilen Methylacrylat, 25 Gew.-Teilen Methylmethacrylat und 10 Gew.-Teilen Methacrylsäure mit 30 % Trockengehalt, entsprechend 89 g Polymersubstanz wurden mit 22,3 g Talkum versetzt und mit 238 g Wasser verdünnt. Die Verarbeitung erfolgte wie Beispiel 1 unter den dort beschriebenen Bedingungen. Die Sprühgeschwindigkeit betrug 4,5 g pro Minute und es ergab sich damit eine Sprühzeit von 124 min. Die überzogenen Tabletten wurden wie in Beispiel 1 nachgetrocknet und getestet. Sie waren 2 Std. magensaftresistent, lösten sich nach jeweils 60 min nicht in Pufferlösungen pH 5,0 und 6,8 und zerfielen in Pufferlösungen pH 7,5 innerhalb von 50 min unter Auflösung des Methylenblau-Farbstoffes.

### Beispiel 3 - Vergleichspolymerisat V1

Wie in Beispiel 1 beschrieben, wurde ein Emulsionspolymerisat aus 50 Gew.-Teilen Methylacrylat, 20 Gew.-Teile Methylmethacrylat und 30 Gew.-Teile Methacrylsäure verarbeitet unter Zusatz von 8,9 g Triethylcitrat als Weichmacher. Die überzogenen Tabletten waren 2 Std. magensaftresistent, waren danach 60 min unverändert in Pufferlösung pH 5,0 und zerfielen in Pufferlösung pH 6,5 innerhalb von 1 Std.

### Beispiel 4 - Überzugsmittel B4

Auf 1 kg kugelige Partikeln mit einem Durchmesser von 0,8-1,2 mm, enthaltend 4,4 Gew.-% Bisacodyl als Wirkstoff, wurden in einem Wirbelschichtgerät GPC der Fa. Glatt GmbH, D-79589 Binzen unter ständigem Wirbeln mit einer Mischung aus 417 g eines 30 %igen Emulsionspolymerisates aus 70 Gew.-Teilen Methylacrylat, 20 Gew.-Teilen Methylmethacrylat und 10 Gew.-Teilen Methacrylsäure und einer Emulsion von 3,8 g Glycerinmonostearat in 224 g Wasser besprüht. Die Sprühgeschwindigkeit betrug 10 g pro min, wobei die Sprühdispersion mittels einer Schlauchpumpe kontinuierlich an die Sprühdüse des Wirbelschichtgerätes transportiert wurde. Die überzogenen Pellets wurden nach USP XXII Apparatur 2 (paddle) auf Magensaftresistenz und Auflösungsgeschwindigkeit in Pufferlösung pH 6,8 geprüft. Die Freisetzung von Bisacodyl aus den in Magensaft nicht zerfallenden Pellets betrug nach 2 Std. weniger als 3 %. Nach Wechsel der Prüfflüssigkeit auf Pufferlösung pH 6,8 zerfielen die Pellets allmählich und gaben innerhalb von 45 min über 99 % des enthaltenden Wirkstoffes frei.

## Patentansprüche

1. Überzugs- und Bindemittel für Arzneiformen, enthaltend ein Copolymerisat aus äthylenisch ungesättigten, radikalisch polymerisierbaren Monomeren mit einer Carboxylgruppe und Alkylestern solcher Monomerer,
**dadurch gekennzeichnet,**
**daß** das Copolymerisat aus
A) 10 bis 25 Gew.-% Methacrylsäure,
B) 40 bis 70 Gew.-% Methylacrylat
C) 20 bis 40 Gew.-% Methylmethacrylat
aufgebaut ist.

2. Überzugs- und Bindemittel für Arzneiformen gemäß Anspruch 1, **dadurch gekennzeichnet, daß** das Copolymerisat zu mehr als 80 Gew.-% aus den Bestandteilen A, B und C aufgebaut ist.

3. Überzugs- und Bindemittel für Arzneiformen gemäß Anspruch 1 oder 2, **dadurch gekennzeichnet, daß** das Copolymerisat als Latex in einer wäßrigen Phase dispergiert vorliegt.

4. Verwendung einer wäßrigen Dispersion des Copolymerisats gemäß Anspruch 3 als wäßriges Überzugs-und Bindemittel für Arzneiformen.

5. Arzneiform, enthaltend ein Überzugs- und/oder Bindemittel aus dem Copolymerisat gemäß Anspruch 1 oder 2.

## Claims

1. Coating and binding agent for pharmaceutical preparations, containing a copolymer of ethylenically unsaturated, radically polymerisable monomers with a carboxyl group and alkyl esters of such monomers, **characterised in that** the copolymer is made up of
A) 10 to 25 wt.% of methacrylic acid,
B) 40 to 70 wt.% of methyl acrylate,
C) 20 to 40 wt.% of methyl methacrylate.

2. Coating and binding agent for pharmaceutical preparations according to claim 1, **characterised in that** more than 80% by weight of the copolymer is made up of components A, B and C.

3. Coating and binding agent for pharmaceutical preparations according to claim 1 or 2, **characterised in that** the copolymer is present as a latex, dispersed in an aqueous phase.

4. Use of an aqueous dispersion of the copolymer according to claim 3 as an aqueous coating and binding agent for pharmaceutical preparations.

5. Pharmaceutical preparation containing a coating and/or binding agent comprising the copolymer according to claim 1 or 2.

## Revendications

1. Compositions d'enrobage et de liant pour des formes médicamenteuses contenant un copolymérisat à base de monomères polymérisables par voie radicalaire, non saturés éthyléniquement, ayant un groupe carboxyle ainsi que les esters d'alkyle de ces monomères,
**caractérisées en ce que**
le copolymérisat est constitué de :
A) 10 à 25 % en poids d'acide méthacrylique
B) 40 à 70 % en poids d'acrylate de méthyle
C) 20 à 40 % en poids de méthacrylate de méthyle.

2. Compositions d'enrobage et de liant pour formes médicamenteuses conformément à la revendication 1,
**caractérisées en ce que**
le copolymérisat est constitué pour plus de 80 % en poids des constituants A, B et C.

3. Compositions d'enrobage et de liant pour formes médicamenteuses conformément à l'une quelconque des revendications 1 ou 2,
**caractérisées en ce que**
le copolymérisat se présente dispersé sous forme de latex dans une phase aqueuse.

4. Utilisation d'une dispersion aqueuse de copolymérisat conformément à la revendication 3, comme composition aqueuse d'enrobage et de liant, pour des formes médicamenteuses.

5. Forme médicamenteuse contenant une composition d'enrobage et de liant à base du copolymérisat conformément à la revendication 1 ou la revendication 2.
